# EUROPEAN PATENT APPLICATION

(11) **EP 0 951 881 A2**
(43) Date of publication of application: **27.10.1999**
(21) Application number: 99303071.7
(22) Date of filing: 21.04.1999
(51) Int. Cl.: A61F 5/44

(54) **External catheter**

(30) Priority: 21.04.1998 GB 9808482
(71) Applicant: Waight, Montague Crichton Every, Newhaven, Sussex BN9 9EJ (GB)
(72) Inventor: Waight, Montague Crichton Every, Newhaven, Sussex BN9 9EJ (GB)
(74) Representative: BROOKES & MARTIN

(57) **Abstract**

An external catheter comprising:
i. a body 1 having an opening against or in which a user's pudenda are receivable,
ii. the body 1 having a sump, and
iii. a conduit 4 for draining the sump into a receiver 20.

## Description

### Field of the Invention

This invention relates to external catheters for use by either sex.

### Background

Many people suffer from full bladders at times where it is impossible, inconvenient or undesirable to use a lavatory. Examples of the first are those bed-ridden. Examples of the second include the infirm who while sufficiently able bodied to rise from bed would find it an effort. Examples of the third include situations where public lavatories are perceived as threatening or unsavoury. In particular a user may not wish to sit on a dirty lavatory. Additionally flushing a lavatory uses large quantities of purified water which is rather extravagant.

### Discussion of the Prior Art

External catheters for non-medical use have been devised. A good example is GB 2 239 804 (Munson and Munson). This arrangement has a number of problems. First it is suitable only for male use. Secondly the device is rather indiscreet. US 4 022 213 (Stein) describes a device somewhat similar to that disclosed in GB 2 239 804 but intended for long term use. Again the device is suitable only for male use. It is provided as part of a garment and is made of flaccid material. US 5 618 277 (Goulter) and WO 97/14353 (Goulter) provide a further examples of catheters for long term male use. They comprise a thin portion provided with a non return valve draining into a urine collection chamber. US 4 202 057 (Ibarra) describes a bed-pan for bedridden women. It comprises an elliptical body with an opening on the top face thereof. WO 90/00 379 (Washington) describes an external catheter for women. It comprises a soft flexible body with central opening. US 5 300 052 describes a urine collector for men. It comprises a soft flexible pouch containing a compartment with hydrophobic beads. It is intended for long term use and by males only.

The invention seeks to reduce the problems associated with the prior art.

### Statement of the Invention

According to the invention there is provided a self supporting external catheter for use by either sex, the catheter comprising:
i. a body having an opening defined by walls directed towards the inside of the catheter body against or in which a user's pudenda are receivable,
ii. the body having a sump,
iii. a drain hole for draining the sump, and
iv. the body comprising two walls tapering to a join opposite the opening.

Generally the body is self supporting. It may be made of rigid material but it is more preferable to make it of resilient but self supporting material for example rubber. The opening may be surrounded by a resilient member. The sump may be defined by walls of the body extending downwardly of the opening. The conduit may comprise flexible piping extending from a drain hole of the sump to the receiver. The receiver may comprise a sealable container.

### Brief Discussion of the Figures

Embodiments of the invention will be described by way of nonlimiting example by reference to the accompanying figures of which :

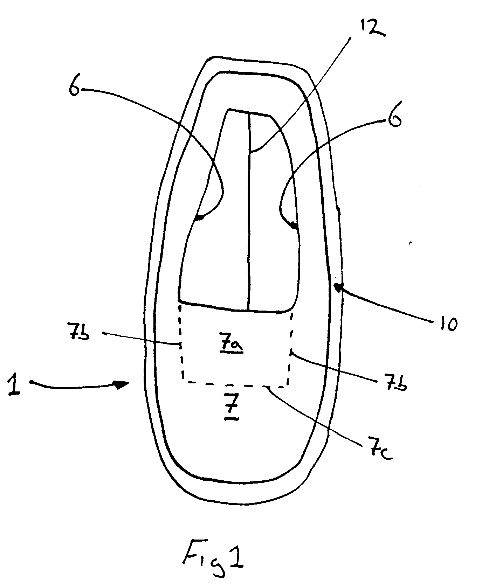

Figure 1 is front elevation of a body of an embodiment;

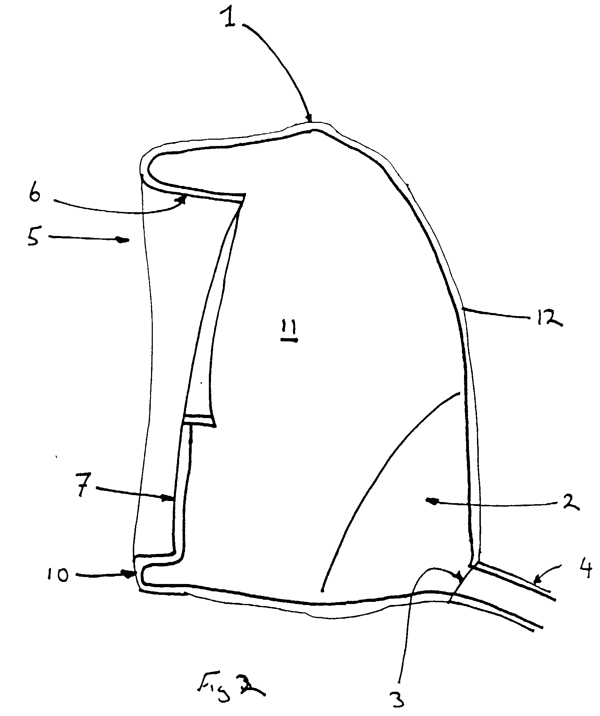

Figure 2 is cross sectional side view of the embodiment;

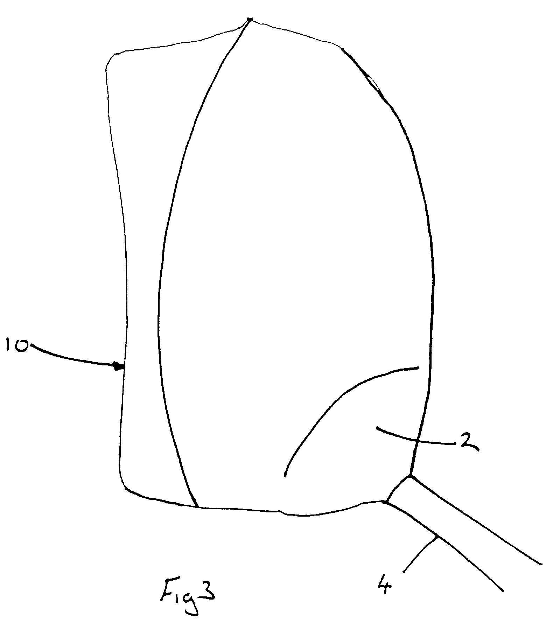

Figure 3 is a side elevation of the embodiment;

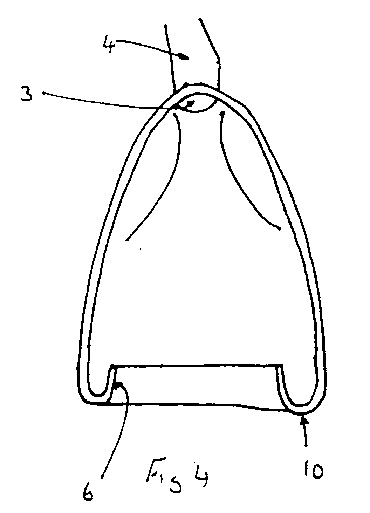

Figure 4 is a longitudinal section;

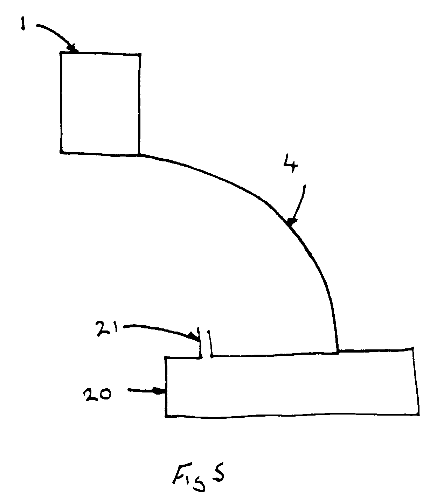

Figure 5 is a schematic of a further embodiment;
Figure 6 is a schematic of a still further embodiment;
Figure 7 is a schematic of a yet further embodiment.
Figure 8 is a scrap section of an embodiment; and

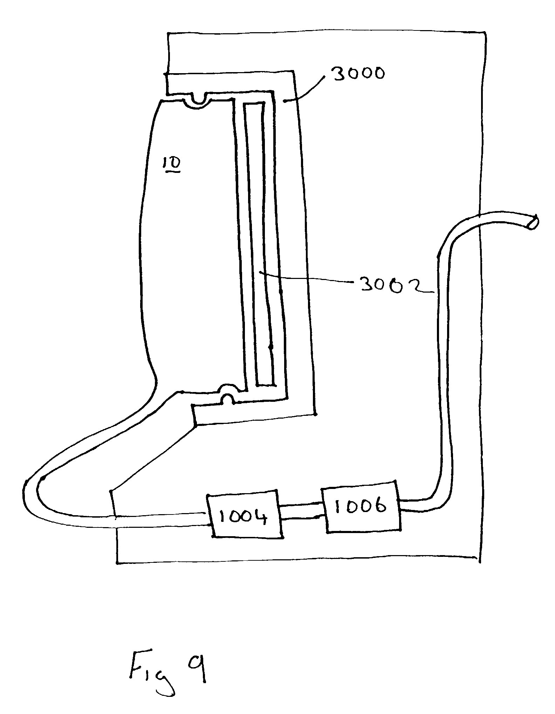

Figure 9 is a schematic of a unit.

### Description of Preferred Embodiments

Body 1 is generally self supporting. Handgrip 2 for example in the form of an arcuate recess may be provided. At or about the base of the body drain hole 3 is provided. A conduit 4 leads from the drain hole 3 to a waste receptacle to be described hereinafter in greater detail.

Front face 5 of the body is provided with an opening defined by inwardly directed walls 6. Inwardly directed walls 6 reduce the risk of splashing. Preferably inwardly directed walls are rigid. Preferably inwardly directed walls 6 are not urged towards each other by resilient biasing. Beneath the walls 6 is provided a sump wall 7. Sump wall 7 is preferably recessed into the body as described hereinafter. Sump wall 7 and side walls of the body define a sump. This feature is advantageous because it provides a reservoir reducing the likelihood of overflowing the event that temporarily the rate of urine inflow exceeds the rate at which it exits the body via drain hole 3. The arrangement also reduces the likelihood of urine contacting the body which reduction is desirable on health grounds. Inwardly directed walls 6 are spaced apart from the outer walls of the body are broadly parallel with the body walls. The inward direction is thus toward the interior of the device.

The device is further provided with pudenda engaging portion 10. In the illustrated embodiment in side elevation pudenda engaging portion 10 is gently arcuate and in front view broadly elliptical. This allows portion 10 to conform closely to the body. A hermetic seal is neither required nor especially desirable. It may be preferred to make portion 10 of resilient material such as rubber or foam to improve comfort in use. If intended for re-use the resilient material may be washable.

Side walls 11 taper inwardly from the opening toward an edge 12. This reduces the propensity of urine to "bounce" back toward the opening which might occur if a flat face was provided perpendicular to the incoming urine flow. The arrangement is also rather more discreet in use since the legs can be closer together. The precise angle is not of importance but the included angle can be in the range of about 30° to about 60° preferably about 40° to about 50° more preferably about 45°. Preferably walls 11 are arcuate. This is preferred both on the ground that the body is stronger but also on the ground that urine deflection can be more efficient. In some cases the side walls meet at an end wall which is about opposite the opening. Provided that the end wall is not large little urine will be deflected back.

As previously noted, the device can be provided with a conduit 4 extending from the drain hole 3. Conveniently this conduit 4 will be an elastomeric material. In some embodiments the conduit 4 simply tenninates leaving a depending free end. This embodiment is particularly intended for use by the mobile in public lavatories. Conduit 4 is not essential for the purpose of the invention. Standing users may find that a suitable stream emerges from the drain hole. A device without the conduit may be more compact, easier to clean and cheaper than one with. The free end of the conduit 4 can be placed in the lavatory and used as described hereinafter. The user need not thus touch the lavatory which may not be clean and touches only the apparatus of the invention of known cleanliness. In other embodiments the conduit 4 discharges into a receiver 20. Receiver 20 can simply be a bag or box provided with a vent 21. It may be desirable to provide sealing means for sealing the receiver for disposal after use. The body and conduit may be disposable or may be cleaned for reuse. In some embodiments of the invention absorbent material can be contained in the receiver. Those skilled will have no difficulty in devising suitable absorbent material for examples those of use in diapers or sanitary towels. It may be desirable to provide receiver 20 and indeed other parts with bacteriostatic materials and/or perfumes.

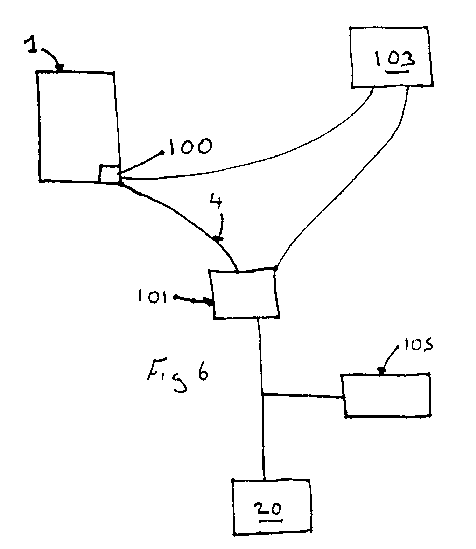

In some embodiments of the invention a pump is provided to allow the receiver or some of the conduit to be above the receiver. The pump can be continuously running or can be activated by a simple switch. A preferred embodiment however is shown in Fig 6. This embodiment is substantially as hereinbefore described. A temperature sensor 100 is provided for sensing the temperature in the flow path. When the sensor 100 senses that the temperature has risen above a certain point the pump 101 is activated. The pump 101 may pump until no more liquid is being pumped or may pump for a period or until the temperature has dropped. The temperature sensor 100 can be mounted outside the body since heat is rapidly conducted through the body walls. In other embodiments the temperature sensor is situated inside the body or the conduit. Those skilled will have little difficulty in devising suitable temperature sensors. A good example is a thermistor the resistance of which is a function of its temperature. Another example is the thermostat. Those skilled will have little difficulty in devising a suitable control circuit 103.

In a further embodiment of the invention switching of the pump can be activated by a flow sensor for example an impeller in the flowline.

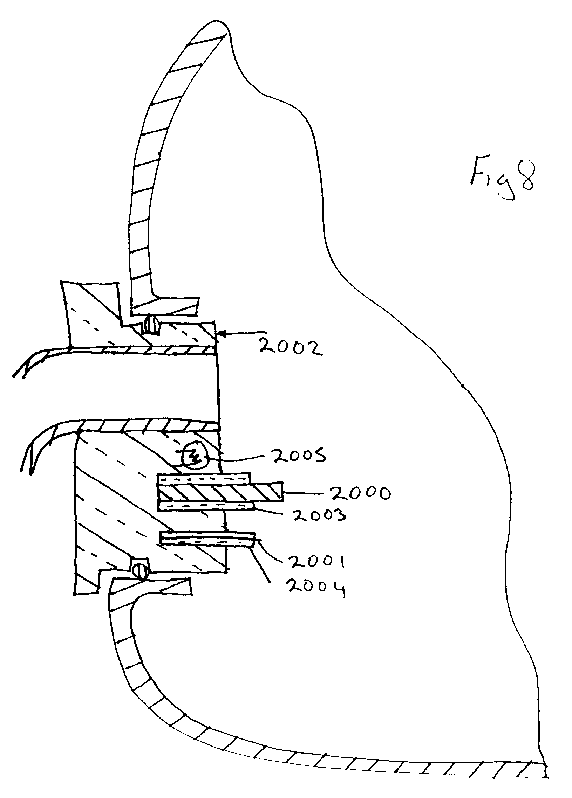

Other sensors are within the scope of the invention. A preferred one is shown in Figure 8. It comprises first and second electrodes 2000, 2001 positioned near the drain. In some embodiments of the invention the body will be disposable. The sensor is generally not disposable. It is provided on the drain tubing 2002. Drain tubing 2002 is in some embodiments not circular at this point and the hole in the body a corresponding shape. This means that the tubing can be arranged to fit the body in one orientation thereby assuring the electrodes are positioned below the drain hole allowing the pump to be actuated promptly. A small potential difference can be put across the electrodes. Current flows across them when contacted by electrolyte rich urine. This current flow is detected by a control circuit and the pump actuated. A number of problems can occur. First a degree of electrolysis can occur at the electrodes and a non-conducting film can be formed. This problem can be substantially reduced by making the electrodes of a stainless steel such Aisi 316. Secondly as the device drains small amounts of urine may remain and the water may evaporate leaving a salt film. This salt film may be sufficiently conducting as to actuate the pump. This problem can be substantially reduced by providing a non conducting preferably hydrophobic sheath 2003, 2004 about the electrodes. A suitable hydrophobic material is PTFE for example monoaxially stretched and hence heat-shrinking PTFE.

In some embodiments of the invention the body is made of transparent or translucent material. When this is done a light can be provided such as encapsulated lamp 2005. Lighting of lamp 2005 illuminates the device with the body acting as a light pipe. This can substantially reduce user's concerns that the device is not correctly positioned.

It will be apparent to the skilled that samples of urine can be drawn off for analysis and that be providing sample and automated analyser 105 urine analysis can be conducted with little human intervention.

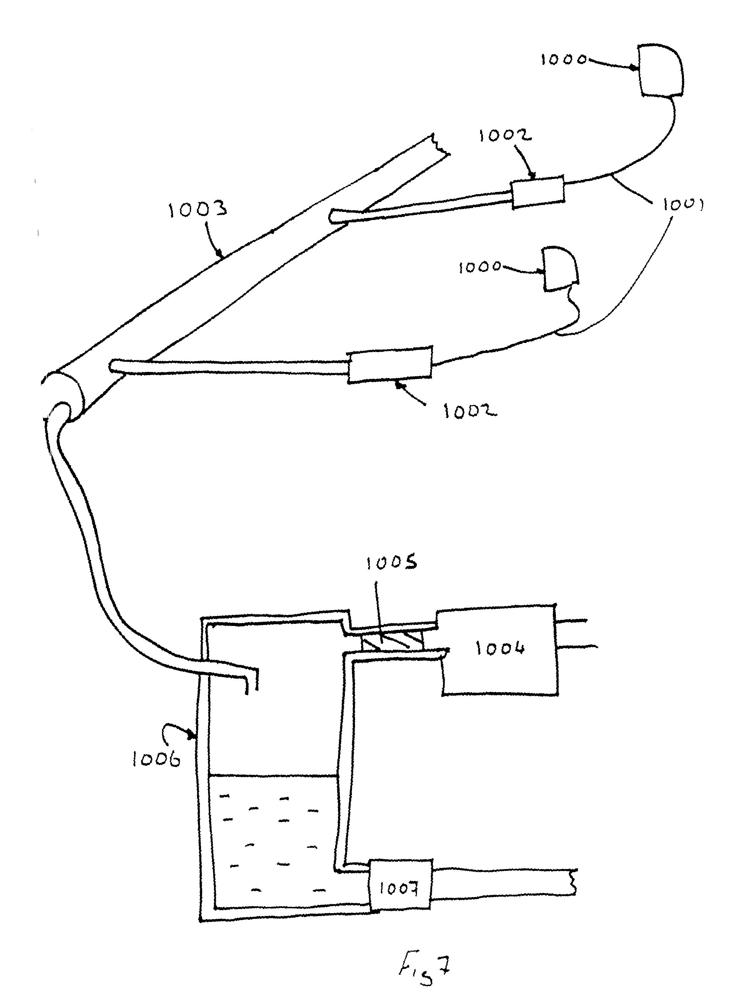

Fig. 7 illustrates a system for multi-station for example hospital use. A plurality of bodies 1000 of the invention are connected by conduit 1001 via valve for example solenoid valve 1002 to main collector 1003. Pump 1004 may reduce the air pressure in the main collector aiding urine transport. A filter 1005 may prevent odours or micro-organisms escaping. Chamber 1006 collects urine for discharge on opening of valve 1007.

In some embodiments of the invention a face 3000 complementary with and engagable by the portion 10 is provided on unit 3001 which includes a pump and store. A detent is preferably provided. This comprises a convenient rest position for the body. Face 3000 may be disposable for hygiene. An absorbent pad 3002 may be provided in face 3000. In some embodiments of the invention detachment of the body from the face can be detected and the light or pump actuated.

Unit 3001 may be provided with outlet 3003 for discharging stored urine for example under control of a pump. Preferably outlet is at least about 40cm for example about 50cm above the ground. This allows easy discharge into sluices or lavatories without excessive splashing. Heights greater than about 70cm preferably about 60cm are not preferred because of the danger of splashing.

Use of the apparatus is straightforward and, unusually for external catheters not particularly gender specific. The user places the opening over the pudenda. In the case of males care is taken to ensure the penis is inserted in the opening defined by walls 6. The user presses the device firmly to the body and urinates into the device. Urine flows through the conduit to waste. As noted the sump provides a temporary reservoir in the event inward flow exceeds outward flow. In some embodiments of the invention a panel 7a is defined by a frangible link 7b on the sump wall 7. Removal of this panel can make access for women user's easier.

The device has many advantages. It may be cheap enough to be disposable after one or a few uses. It allows unclean public lavatories to be used without risk of infection. It is usable by either sex. The device of the invention is much easier and more comfortable to use than a conventional bed pan.

In some embodiments of the invention a panel 7a is defined by a frangible link on the sump wall. In some embodiments of the invention the panel is completely defined by frangible link side lines 7b and base line 7c. In other embodiments base line 7c is omitted and may be replaced by a fold line. Removal of the panel 7a can make access for women user's easier. Rupture of the frangible sidelines allows the panel to be folded into the body. This also allows easier access for women user's. Furthermore it provides a penis support for long term use by men.

## Claims

1. An external catheter comprising:
a body having an opening defined by walls, the body having a sump and a drain hole for draining the sump.
characterised in that the catheter is usable by either sex, in that the body is self supporting and in that the body comprises two walls tapering to an abutment opposite the opening.

2. An external catheter as claimed in claim 1 wherein the two walls taper to abut at a join line or a wall opposite the opening.

3. An external catheter as claimed in claim 1 or claim 2 wherein walls directed to the interior of the body when the catheter is not in use are provided.

4. An external catheter as claimed in any one of the preceding claims wherein a sump wall below the opening is provided with a panel removable or movable to increase the size of the opening is provided.

5. An external catheter as claimed in any one of the preceding claims further comprising a sensor for sensing the presence of fluid in the catheter, a pump for pumping fluid in the catheter and a control circuit for controlling the pump in response to the out put of the sensor.

6. An external catheter as claimed in claim 5 further comprising a drain tube releasably connected to the body, the drain tube comprising a non-cylindrical plug received in a complementary socket of the body, the sensor being mounted on the plug.

7. An external catheter as claimed in claim 6 wherein a light is provided in the plug.

8. An external catheter as claimed in claim 7 the body being removably mounted on a housing and means for actuating the light the means for actuating the lamp being actuated by demounting of the body from the housing.

9. An external catheter as claimed in any one of the preceding claims wherein a sump wall below the opening is recessed into the body.
